# EUROPEAN PATENT APPLICATION

(11) **EP 4 693 308 A1**
(43) Date of publication of application: **11.02.2026**
(21) Application number: 25176820.6
(22) Date of filing: 15.05.2025
(51) Int. Cl.: G16C 20/40, H01M 10/052

(54) **METHOD AND SYSTEM FOR SCREENING MATERIAL FOR LITHIUM SECONDARY BATTERY**

(30) Priority: 08.08.2024 KR 20240106022
(71) Applicant: SAMSUNG SDI CO., LTD., Yongin-si, Gyeonggi-do 17084 (KR)
(72) Inventor: KIM, Soojin, Yongin-si, Gyeonggi-do 17084 (KR); KIM, Dongyoung, Yongin-si, Gyeonggi-do 17084 (KR); KIM, Hyeonsu, Yongin-si, Gyeonggi-do 17084 (KR); KIM, Seulwoo, Yongin-si, Gyeonggi-do 17084 (KR); LEE, Sunmin, Yongin-si, Gyeonggi-do 17084 (KR); SINGH, Jiten, Yongin-si, Gyeonggi-do 17084 (KR); LEE, Kyungmin, Yongin-si, Gyeonggi-do 17084 (KR); SUH, Seungbum, Yongin-si, Gyeonggi-do 17084 (KR)
(74) Representative: Marks & Clerk LLP

(57) **Abstract**

A method and system for screening a material for a lithium secondary battery. The method for screening a material configured to be used in a lithium secondary battery may include: receiving information about an organic substance; generating a database by storing the information about the organic substance based on a plurality of parameters; and applying at least one filter to the database to output information about a target substance configured to be used in a lithium secondary battery among the organic substances, wherein at least one of the target substances is configured to form a solid electrolyte interphase (SEI) layer including an anion of a lithium salt.

## Description

### BACKGROUND

### 1. Field

The present disclosure relates to a lithium secondary battery material screening method and system for outputting information on a target substance that makes the formation of a solid electrolyte interphase (SEI) layer including an anion of a lithium salt.

### 2. Description of the Related Art

While primary batteries are not designed to be (re)charged, secondary (also known as rechargeable) batteries are designed to be discharged and recharged. Among secondary batteries, low-capacity secondary batteries are widely used in portable, small electronic devices, such as smart phones, feature phones, notebook computers, digital cameras, and camcorders, while high-capacity secondary batteries are widely used as power sources for driving motors in hybrid vehicles and electric vehicles, as well as for storing power (e.g., home and/or utility scale power storage). A secondary battery generally includes an electrode assembly including a positive electrode and a negative electrode, a case accommodating both electrodes, and electrode terminals connected to the electrode assembly.

A secondary battery typically includes an electrolyte to allow lithium ions to migrate within. Various materials may be used as the electrolyte, but it is desirable to replace the electrolyte materials previously used in the electrolyte with other replacement electrolyte materials due to reasons such as environmental, process safety, and yield improvement, etc. For example, the secondary battery may deteriorate and result in having a reduced lifespan as it continues to be used; accordingly, it is desirable to replace the electrolyte material in the secondary battery to suppress deterioration.

Finding candidate materials configured to be used in the electrolyte is experimentally time consuming, and requires a great degree of effort and resources. In fact, finding candidate electrolyte materials meeting their requisite conditions is a great challenge for secondary battery manufacturers.

This Background section is for of the general understanding of the background of the present disclosure, and therefore, it may contain information that does not constitute related (or prior) art.

### SUMMARY

It is an object of the present disclosure to provide a solution to the above-mentioned technical challenges.

An aspect of the present disclosure relates to a method and system for screening a material for a lithium secondary battery.

These and other aspects and features of the present disclosure will be described in or will be apparent from the following description of embodiments of the present disclosure.

According to an embodiment of the present disclosure, a method for screening a material for a lithium secondary battery may include: receiving information about an organic substance; generating a database by storing the information about the organic substance based on a plurality of parameters; and applying at least one filter to the database to output information about a target substance configured to be used in a lithium secondary battery among the organic substances, wherein at least some of the target substances may make the formation of at least of a solid electrolyte interphase (SEI) layer including an anion of a lithium salt.

According to another embodiment of the present disclosure, a method for screening a material configured to be used in a lithium secondary battery, comprises: receiving information about one or more organic substances; generating a database by storing the information about the one or more organic substances based on a plurality of parameters; and applying at least one filter to the database to generate output information about one or more target substances configured to be used in the lithium secondary battery, the one or more target substances selected from the one or more organic substances, wherein at least one of the one or more target substances is configured to form a solid electrolyte interphase (SEI) layer comprising an anion of a lithium salt.

According to an embodiment of the present disclosure, the plurality of parameters may include parameters associated with at least one of an ion type, a molecular weight, an electron affinity, an ionization energy, a lithium ion (Li⁺) reaction energy, or a coordination energy between an organic substance and an anion of a lithium salt.

According to another embodiment of the present disclosure, the plurality of parameters may comprise an ion type, a molecular weight, an electron affinity, an ionization energy, a lithium ion (Li⁺) reaction energy, and/or a coordination energy between the one or more organic substances and an anion of a lithium salt.

According to an embodiment of the present disclosure, the at least one filter may include a first filter, wherein the first filter may filter out, among the materials included in the database, a material having an electron affinity greater than an electron affinity threshold.

According to another embodiment of the present disclosure, the at least one filter may comprise a first filter, wherein the first filter filters out a candidate material having an electron affinity greater than a predetermined electron affinity threshold.

According to an embodiment of the present disclosure, the electron affinity threshold may be associated with an electron affinity of a representative organic solvent which is an organic solvent of the lithium secondary battery.

According to another embodiment of the present disclosure, the predetermined electron affinity threshold may be related to an electron affinity of an organic solvent previously determined to be used in the lithium secondary battery.

According to an embodiment of the present disclosure, the at least one filter may include a second filter, wherein the second filter may filter out, among the materials included in the database, a material having an ionization energy greater than an ionization energy threshold.

According to another embodiment of the present disclosure, the at least one filter may comprise a second filter, wherein the second filter filters out a candidate material having an ionization energy greater than a predetermined ionization energy threshold.

According to an embodiment of the present disclosure, the ionization energy threshold may be associated with an internal environment of the lithium secondary battery.

According to another embodiment of the present disclosure, the predetermined ionization energy threshold may be related to an internal environment of the lithium secondary battery.

According to an embodiment of the present disclosure, the at least one filter may include a third filter, wherein the third filter may filter out, among the materials included in the database, a material having a lithium ion reaction energy less than a lithium ion reaction energy threshold.

According to another embodiment of the present disclosure, the at least one filter may comprise a third filter, wherein the third filter filters out a candidate material having a lithium ion reaction energy less than a predetermined lithium ion reaction energy threshold.

According to an embodiment of the present disclosure, the lithium ion reaction energy threshold may be associated with a reaction energy between a lithium ion and a representative additive which is an electrolyte additive of the lithium secondary battery.

According to another embodiment of the present disclosure, the predetermined lithium ion reaction energy threshold may be related to a reaction energy between a lithium ion and an electrolyte additive previously determined to be used in the lithium secondary battery.

According to an embodiment of the present disclosure, the at least one filter may include a fourth filter, wherein the fourth filter may filter out, among the materials included in the database, a material having a coordination energy between the material included in the database and a hexafluorophosphate ion (PF₆⁻) less than a hexafluorophosphate ion coordination energy threshold.

According to another embodiment of the present disclosure, the at least one filter may comprise a fourth filter, wherein the fourth filter filters out a candidate material having a coordination energy between the candidate material and a hexafluorophosphate ion (PF₆⁻) less than a predetermined hexafluorophosphate ion coordination energy threshold.

According to an embodiment of the present disclosure, the hexafluorophosphate ion coordination energy threshold may be associated with a coordination energy between a hexafluorophosphate ion and a representative organic solvent which is an organic solvent of the lithium secondary battery.

According to another embodiment of the present disclosure, the predetermined hexafluorophosphate ion coordination energy threshold may be related to a coordination energy between the hexafluorophosphate ion and an organic solvent previously determined to be used in the lithium secondary battery.

According to an embodiment of the present disclosure, the representative organic solvent may be one of ethylene carbonate (EC), fluoroethylene carbonate (FEC), ethylmethyl carbonate (EMC), dimethyl carbonate (DMC), ethyl propionate (EP), and ethyl acetate (EA).

According to another embodiment of the present disclosure, the organic solvent may comprise ethylene carbonate (EC), fluoroethylene carbonate (FEC), ethylmethyl carbonate (EMC), dimethyl carbonate (DMC), ethyl propionate (EP), or ethyl acetate (EA).

According to an embodiment of the present disclosure, the representative additive may be one of vinylene carbonate (VC) and fluoroethylene carbonate (FEC).

According to another embodiment of the present disclosure, the electrolyte additive may comprise vinylene carbonate (VC) or fluoroethylene carbonate (FEC).

According to an embodiment of the present disclosure, the method for screening a material for a lithium secondary battery may further include: receiving an input for selecting at least one of the plurality of parameters, and receiving information about a numerical range for the selected parameter, wherein the at least one filter may include a fifth filter, wherein the fifth filter may filter out, among the materials included in the database, a material in which information related to at least one of a plurality of selected parameters of the materials included in the database is included in the numerical range.

According to another embodiment of the present disclosure, the method may further include: receiving an input for selecting at least one of the plurality of parameters; and receiving information about a numerical range for the at least one of the plurality of parameters, wherein the at least one filter comprises a fifth filter, and wherein the fifth filter filters out a candidate material in which the numerical range comprises information related to the at least one of the plurality of parameters.

According to an embodiment of the present disclosure, the outputting information about a target substance may include receiving an input of selecting at least one of the plurality of parameters on a user interface, and outputting information about at least one of the plurality of parameters selected among information about the target substance on the user interface.

According to another embodiment of the present disclosure, the generating of the output information may comprise: receiving an input of selecting at least one of the plurality of parameters on a user interface; and generating output information about the at least one of the plurality of parameters selected from the output information about the one or more target substances on the user interface.

There may be provided a computer program stored in a computer-readable recording medium for executing the method according to an embodiment of the present disclosure on a computer.

According to an embodiment of the present disclosure for solving the above technical problems, an information processing system may include: a communication module, a memory, and at least one processor connected to the memory and configured to execute at least one computer-readable program included in the memory, wherein the at least one program may include instructions for: receiving information about an anion of a lithium salt, receiving information about an organic substance, generating a database by storing the information about the anion of the lithium salt and the information about the organic substance based on a plurality of parameters, and applying at least one filter to the database to output information about a target substance configured to be used in a lithium secondary battery among the organic substances, wherein at least some of the target substances may make the formation of at least of an SEI layer including the anion of the lithium salt.

According to another embodiment of the present disclosure, an information processing system may comprise: a communication module; a memory; and at least one processor connected to the memory and configured to execute at least one computer-readable program comprised in the memory, wherein the at least one program comprises instructions for: receiving information about one or more organic substances, generating a database by storing the information about the one or more organic substances based on a plurality of parameters, and applying at least one filter to the database to generate output information about one or more target substances configured to be used in a lithium secondary battery, the one or more target substances selected from the or more organic substances, wherein at least one of the one or more target substances is configured to form a solid electrolyte interphase (SEI) layer comprising an anion of a lithium salt.

According to an embodiment of the present disclosure, the at least one filter may include a first filter, wherein the first filter may filter out, among the materials included in the database, a material having an electron affinity greater than an electron affinity threshold.

According to another embodiment of the present disclosure, the at least one filter may comprise a first filter, wherein the first filter filters out a candidate material having an electron affinity greater than a predetermined electron affinity threshold.

According to an embodiment of the present disclosure, the at least one filter may include a second filter, wherein the second filter may filter out, among the materials included in the database, a material having an ionization energy greater than an ionization energy threshold.

According to another embodiment of the present disclosure, the at least one filter may comprise a second filter, wherein the second filter filters out a candidate material having an ionization energy greater than a predetermined ionization energy threshold.

According to an embodiment of the present disclosure, the at least one filter may include a third filter, wherein the third filter may filter out, among the materials included in the database, a material having a lithium ion reaction energy less than a lithium ion reaction energy threshold.

According to another embodiment of the present disclosure, the at least one filter may comprise a third filter, the third filter filters out a candidate material having a lithium ion reaction energy less than a predetermined lithium ion reaction energy threshold.

According to an embodiment of the present disclosure, the at least one filter may include a fourth filter, wherein the fourth filter may filter out, among the materials included in the database, a material having a coordination energy between the material included in the database and a hexafluorophosphate ion less than a hexafluorophosphate ion coordination energy threshold.

According to another embodiment of the present disclosure, the at least one filter may comprise a fourth filter, wherein the fourth filter filters out a candidate material having a coordination energy between the candidate material and a hexafluorophosphate ion (PF₆⁻) less than a predetermined hexafluorophosphate ion coordination energy threshold.

According to various embodiments of the present disclosure, when a name capable of specifying an organic substance is input into a lithium secondary battery material screening system, the lithium secondary battery material screening system can generate information about the organic substance and automatically build a database based on the information about the organic substance.

According to various embodiments of the present disclosure, it is possible to easily search for an organic substance configured to be used in a secondary battery using a database without any separate experiments. In addition, it may take a lot of time and effort to find a material that suppresses deterioration of a secondary battery, but since it is not necessary to perform repeated experiments to find the material (e.g., an organic substance) that suppresses the deterioration of the secondary battery, time, effort, resources, costs, etc. can be saved. For example, by obtaining information about an organic substance that is stable to hexafluorophosphate ion (PF₆⁻) in a secondary battery including a lithium iron phosphate (LFP), an organic substance that slows down or suppresses deterioration of a secondary battery including the lithium iron phosphate can be easily searched.

According to various embodiments of the present disclosure, a material that suppresses deterioration of a lithium secondary battery caused by an anion of a lithium salt among organic substances can be screened using a plurality of filters.

According to various embodiments of the present disclosure, a filter can be easily created through a user interface, and a target substance that suppresses deterioration of a lithium secondary battery can be easily searched using the created filter.

According to various embodiments of the present disclosure, an organic substance suitable for various conditions of a lithium secondary battery can be searched by a user selecting at least one of a plurality of parameters and inputting a numerical range for the selected parameter to create a filter.

According to various embodiments of the present disclosure, information about a target substance can be displayed with high readability through a user interface. A more suitable substance can be selected by a user comparing substances included in the target substances with each other through the user interface.

However, aspects and features of the present disclosure are not limited to those described above, and other aspects and features not mentioned will be clearly understood by a person skilled in the art from the detailed description, described below. At least some of the above and other features of the invention are set out in the claims.

### BRIEF DESCRIPTION OF THE DRAWINGS

The drawings illustrate embodiments of the present disclosure, and further describe aspects and features of the present disclosure along with the detailed description of the present disclosure. Thus, the present disclosure should not be construed as being limited to the drawings.
FIG. 1 is a schematic diagram showing a lithium secondary battery material screening system, according to an embodiment of the present disclosure.
FIG. 2 is a schematic diagram showing a configuration in which an information processing system is connected to and communicates with a plurality of user terminals for screening a lithium secondary battery material, according to an embodiment of the present disclosure.
FIG. 3 is a block diagram showing an internal configuration of a user terminal and an information processing system, according to an embodiment of the present disclosure.
FIG. 4 is a cross-sectional diagram showing a lithium secondary battery, according to an embodiment of the present disclosure.
FIG. 5 is a schematic diagram showing deterioration of a lithium secondary battery including a positive electrode, a negative electrode, and an electrolyte, according to one embodiment of the present disclosure.
FIG. 6 is a schematic diagram showing a lithium secondary battery including a positive electrode, a negative electrode, and an electrolyte, according to an embodiment of the present disclosure.
FIG. 7 is shows suitable organic substances, according to an embodiment of the present disclosure.
FIG. 8 is a flowchart showing an example of a lithium secondary battery material screening method, according to an embodiment of the present disclosure.
FIG. 9 is a schematic diagram showing generating output information about the target substance using a plurality of filters, according to an embodiment of the present disclosure.
FIG. 10 is a schematic diagram showing an example of a user interface having output information about a target substance, according to an embodiment of the present disclosure.
FIG. 11 is a schematic diagram showing an example of a filter input interface, according to one embodiment of the present disclosure.

### DETAILED DESCRIPTION

Hereinafter, embodiments of the present disclosure will be described, in detail, with reference to the accompanying drawings. The terms or words used in this specification and claims should not be construed as being limited to the usual or dictionary meaning and should be interpreted as meaning and concept consistent with the technical idea of the present disclosure based on the principle that the inventor can be his/her own lexicographer to appropriately define the concept of the term to explain his/her invention in the best way.

The embodiments described in this specification and the configurations shown in the drawings are only some of the embodiments of the present disclosure and do not represent all of the technical ideas, aspects, and features of the present disclosure. Accordingly, it should be understood that there may be various equivalents and modifications that can replace or modify the embodiments described herein at the time of filing this application.

It will be understood that when an element or layer is referred to as being "on," "connected to," or "coupled to" another element or layer, it may be directly on, connected, or coupled to the other element or layer or one or more intervening elements or layers may also be present. When an element or layer is referred to as being "directly on," "directly connected to," or "directly coupled to" another element or layer, there are no intervening elements or layers present. For example, when a first element is described as being "coupled" or "connected" to a second element, the first element may be directly coupled or connected to the second element or the first element may be indirectly coupled or connected to the second element via one or more intervening elements.

To facilitate understanding of the disclosure, the attached drawings are not drawn to actual scale and the dimensions of some components may be exaggerated. Furthermore, the same reference numbers may be assigned to the same components in different embodiments. As used herein, the term "and/or" includes any and all combinations of one or more of the associated listed items. Further, the use of "may" when describing embodiments of the present disclosure relates to "one or more embodiments of the present disclosure." Expressions, such as "at least one of" and "any one of," when preceding a list of elements, modify the entire list of elements and do not modify the individual elements of the list. When phrases such as "at least one of A, B and C", "at least one of A, B or C," "at least one selected from a group of A, B and C," or "at least one selected from among A, B and C" are used to designate a list of elements A, B and C, the phrase may refer to any and all suitable combinations or a subset of A, B and C, such as A, B, C, A and B, A and C, B and C, or A and B and C. As used herein, the terms "use," "using," and "used" may be considered synonymous with the terms "utilize," "utilizing," and "utilized," respectively. As used herein, the terms "substantially," "about," and similar terms are used as terms of approximation and not as terms of degree, and are intended to account for the inherent variations in measured or calculated values that would be recognized by those of ordinary skill in the art.

It will be understood that, although the terms first, second, third, etc. may be used herein to describe various elements, components, regions, layers, and/or sections, these elements, components, regions, layers, and/or sections should not be limited by these terms. These terms are used to distinguish one element, component, region, layer, or section from another element, component, region, layer, or section. Thus, a first element, component, region, layer, or section discussed below could be termed a second element, component, region, layer, or section without departing from the teachings of example embodiments.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" or "over" the other elements or features. Thus, the term "below" may encompass both an orientation of above and below. The device may be otherwise oriented (rotated 90 degrees or at other orientations), and the spatially relative descriptors used herein should be interpreted accordingly.

The terminology used herein is for the purpose of describing embodiments of the present disclosure and is not intended to be limiting of the present disclosure. As used herein, the singular forms "a" and "an" are intended to include the plural forms as well, unless the context clearly indicates otherwise. It will be further understood that the terms "includes," "including," "comprises," and/or "comprising," when used in this specification, specify the presence of stated features, integers, steps, operations, elements, and/or components but do not preclude the presence or addition of one or more other features, integers, steps, operations, elements, components, and/or groups thereof.

Also, any numerical range disclosed and/or recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein, and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

References to two compared elements, features, etc. as being "the same" may mean that they are "substantially the same". Thus, the phrase "substantially the same" may include a case having a deviation that is considered low in the art, for example, a deviation of 5% or less. In addition, when a certain parameter is referred to as being uniform in a given region, it may mean that it is uniform in terms of an average.

Throughout the specification, unless otherwise stated, each element may be singular or plural.

Arranging an arbitrary element "above (or below)" or "on (under)" another element may mean that the arbitrary element may be disposed in contact with the upper (or lower) surface of the element, and another element may also be interposed between the element and the arbitrary element disposed on (or under) the element.

In addition, it will be understood that when a component is referred to as being "linked," "coupled," or "connected" to another component, the elements may be directly "coupled," "linked" or "connected" to each other, or another component may be "interposed" between the components".

Throughout the specification, when "A and/or B" is stated, it means A, B or A and B, unless otherwise stated. That is, "and/or" includes any or all combinations of a plurality of items enumerated. When "C to D" is stated, it means C or more and D or less, unless otherwise specified.

Hereinafter, a case in which information on a material (e.g., an electron affinity of a material included in a database) is greater than or less than a threshold (e.g., an electron affinity threshold) is described separately, but the present disclosure is not limited thereto. For example, a configuration for filtering out a material included in a database having an electron affinity greater than an electron affinity threshold may be applied when filtering out a material included in a database having an electron affinity greater than or equal to an electron affinity threshold. Similarly, a configuration for filtering out a material included in a database having an electron affinity less than or equal to an electron affinity threshold may be applied when filtering out a material included in a database having an electron affinity less than an electron affinity threshold.

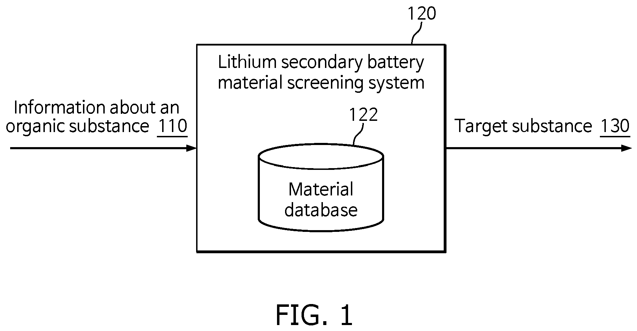

FIG. 1 is a schematic diagram of a lithium secondary battery material screening system 120 according to an embodiment of the present disclosure. The lithium secondary battery material screening system 120 may receive information 110 about an organic substance. Here, the organic substance may be included at least as a part of an electrolyte included in a lithium secondary battery. For example, the organic substance may be used as an organic solvent for an electrolyte included in a lithium secondary battery. For example, the organic substance may be used as an additive for an electrolyte included in a lithium secondary battery.

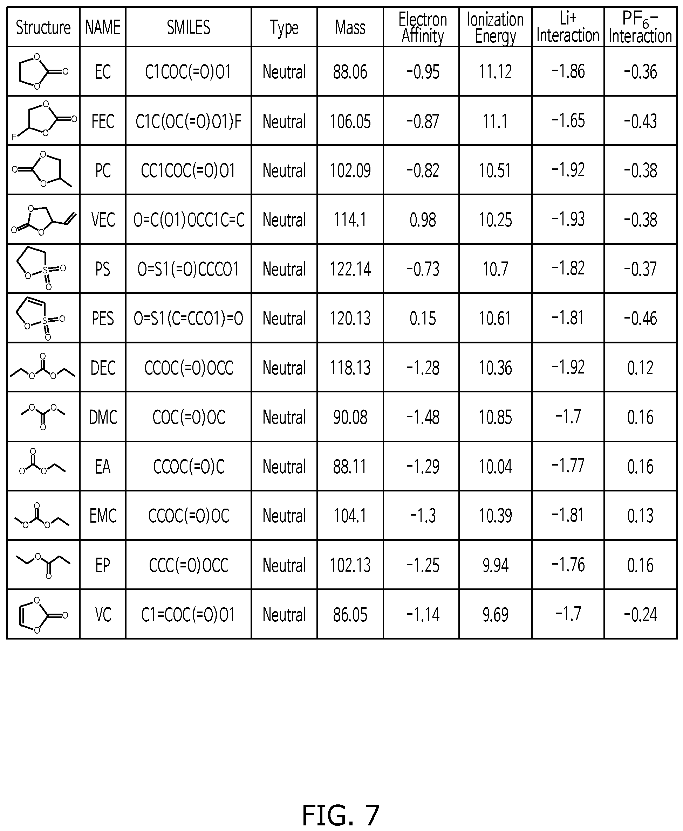

The information 110 about an organic substance may include information about molecular characteristics and/or chemical characteristics of the organic substance. For example, the information 110 about an organic substance may include information related to ion types (e.g., anionic, cationic, neutral, etc.), molecular weight, dipole moment, electron affinity, ionization energy, lithium-ion reaction energy (e.g., interaction energy for the reaction between organic substances and lithium ions), coordination energy between organic substances and anions of lithium salts (e.g., PF₆⁻, BF₄⁻, SbF₆⁻, AsF₆⁻, ClO₄⁻, AlO₂⁻, AlCl₄⁻, PO₂F₂⁻, Cl⁻, I⁻, etc.), highest occupied molecular orbital (HOMO) energy, lowest unoccupied molecular orbital (LUMO) energy, protonation energy (PE), dehydrogenation energy (DE), reaction energy of oxygen radicals, electron affinity of lithium ion complexes containing the organic substances, LUMO of lithium ion complexes containing the organic substances, reaction energy of lithium ion complexes containing the organic substances with oxygen radicals, reaction energy of the organic substances with transition metals (e.g., Ni²⁺, Fe²⁺, Co²⁺, Mn²⁺, etc.), etc. Here, the coordination energy between the organic substance and the anion of the lithium salt refers to a Gibbs free energy change amount for the coordination bond between the organic substance and the anion of the lithium salt. The protonation energy refers to a Gibbs free energy change amount for a reaction in which an organic substance bonds to a proton. The dehydrogenation energy refers to a Gibbs free energy change amount for a reaction in which a bond with a hydrogen atom (or a hydrogen radical or a hydrogen molecule) in an organic substance is dissociated. The reaction energy of an oxygen radical refers to a Gibbs free energy change amount for a reaction in which an organic substance bonds to an oxygen radical. An example of an organic substance and information 110 about the organic substance is described in detail with reference to FIG. 7 and Table 1.

The lithium ion complex including an organic substance refers to a complex in which a lithium ion (Li⁺) and an organic substance are combined. The reaction energy between an oxygen radical and a lithium ion complex including an organic substance refers to a Gibbs free energy change amount according to a reaction in which the oxygen radical combines with the lithium ion complex including an organic substance.

In an embodiment, the lithium salt may be included in an electrolyte for a lithium secondary battery. The lithium salt may be dissolved in an organic solvent included in the electrolyte, act as a source of lithium ions in the lithium secondary battery, enable a basic operation of the lithium secondary battery, and/or promote the migration of the lithium ions between the positive and negative electrodes. For example, the lithium salt may include one or more of LiPF₆, LiBF₄, LiSbF₆, LiAsF₆, LiClO₄, LiAlO₂, LiAlCl₄, LiPO₂F₂, LiCl, Lil, LiN(SO₃C₂F₅)₂, Li(FSO₂)₂N (lithium bis(fluorosulfonyl)imide (LiFSI)), LiC₄F₉SO₃, LiN(CₓF₂ₓ₊₁SO₂)(C_{y}F_{2y+1}SO₂) (x and y are integers of 1 to 20), lithium trifluoromethane sulfonate, lithium tetrafluoroethanesulfonate, lithium difluorobis(oxalato)phosphate (LiDFOB), and lithium bis(oxalato)borate (LiBOB).

In an embodiment, the information 110 about the organic substance may include a name capable of specifying the organic substance. For example, the name capable of specifying the organic substance may include a compound name, a simplified molecular input line entry system (SMILES), a CAS number, etc. In this case, the lithium secondary battery material screening system 120 may generate information 110 on the organic substance including information on molecular characteristics and chemical characteristics of the organic substance based on the name capable of specifying the organic substance. For example, the lithium secondary battery material screening system 120 may specify an organic substance based on the name capable of specifying the organic substance. The lithium secondary battery material screening system 120 may generate a molecular geometry for the specified organic substance using a density functional theory (DFT) calculation to optimize the molecular geometry. The lithium secondary battery material screening system 120 may generate information about molecular characteristics and chemical characteristics of the organic substance based on the molecular geometry for the organic substance. Additionally or alternatively, the lithium secondary battery material screening system 120 may receive at least some of the information about molecular characteristics and chemical characteristics of the organic substance generated from an external system.

In an embodiment, information required to generate information on the molecular characteristics and chemical characteristics of the organic substance may be determined in advance. For example, information on oxygen radicals, information on lithium ions, information on anions of lithium salts, etc. may be determined in advance. Specifically, information on ion types, molecular weights, electron affinities, ionization energies, branching geometries, etc., for oxygen radicals, lithium ions, anions of lithium salts, etc., may be determined in advance. Additionally or alternatively, the lithium secondary battery material screening system 120 may receive information required to generate information on the molecular characteristics and chemical characteristics of the organic substance in advance. In an embodiment, information required to generate information on molecular characteristics and chemical characteristics of organic substances may also be generated using DFT calculations.

The lithium secondary battery material screening system 120 may generate a material database 122 by storing information about organic substances based on a plurality of parameters. The material database 122 may store information on organic substances by classifying them into a plurality of parameters. The plurality of parameters may refer to categories of information about organic substances. For example, the plurality of parameters may include parameters related to ion types, molecular weight, dipole moment, electron affinity, ionization energy, lithium-ion reaction energy, coordination energy between organic substances and anions of lithium salts, HOMO energy, LUMO energy, protonation energy (PE), dehydrogenation energy (DE), reaction energy of oxygen radicals, electron affinity of lithium ion complexes containing organic substances, LUMO of lithium ion complexes containing organic substances, reaction energy of lithium ion complexes containing organic substances with oxygen radicals, reaction energy of organic substances with transition metals, etc.

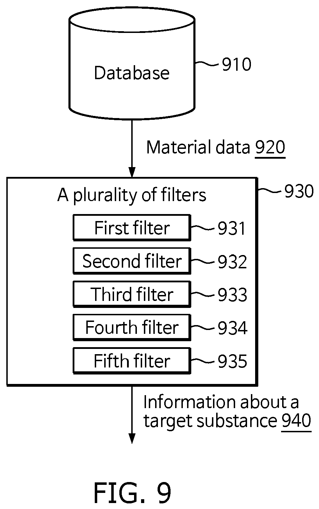

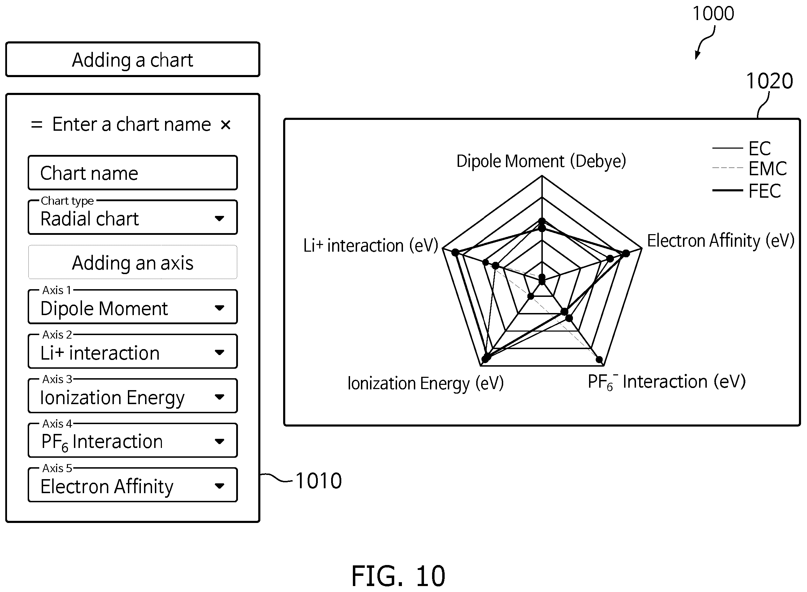

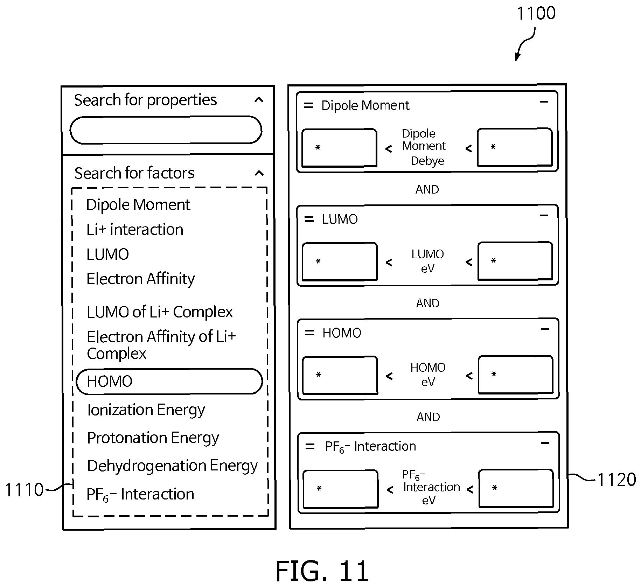

The lithium secondary battery material screening system 120 may generate output information 130 about a target substance configured to be used in a lithium secondary battery among organic substances by applying at least one filter to the material database 122. In a lithium secondary battery including a target substance, the target substance can suppress deterioration of the lithium secondary battery typically caused by the anion of the lithium salt. The lithium secondary battery material screening system 120 may generate output information 130 about the target substance to a user terminal, etc. Additionally, the output information 130 about the target substance may be displayed on a user interface. A schematic of generating output information 130 about the target substance, by applying at least one filter to the material database 122, will be described in detail with reference to FIG. 9, and a user interface having output information 130 about the target substance will be described in detail with reference to FIGS. 10 and 11.

In an embodiment, when a name capable of specifying an organic substance is inputted into the lithium secondary battery material screening system 120, the lithium secondary battery material screening system 120 can generate information 110 about the organic substance, and automatically build a database (e.g., a material database 122) based on the information 110 about the organic substance.

In an embodiment, the organic substance configured to be used in the secondary battery can be easily searched using the database without any separate experiments. It may take a lot of time and effort to find a material that suppresses deterioration of a secondary battery, but because it is not necessary to perform repeated experiments to find a material (e.g., an organic substance) capable of suppressing the deterioration of the secondary battery, time, effort, resources, costs, etc. can be saved. For example, by obtaining information about an organic substance that is stable to hexafluorophosphate ion (PF₆⁻) in a secondary battery including lithium iron phosphate (LFP), an organic substance capable of slowing down or suppressing deterioration of a secondary battery including the lithium iron phosphate can be easily searched.

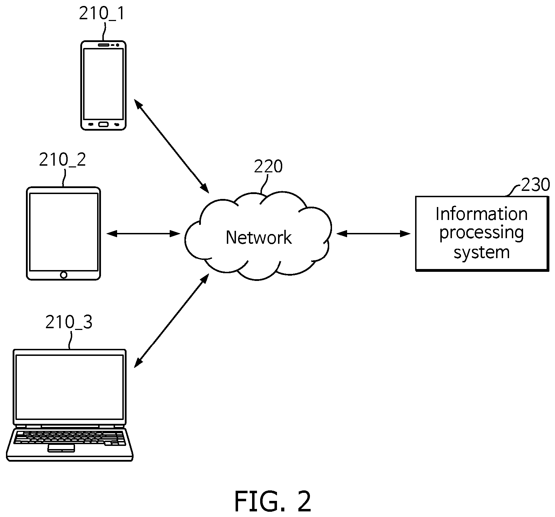

FIG. 2 is a schematic diagram showing a configuration in which an information processing system 230 is connected to and communicates with a plurality of user terminals 210_1, 210_2, and 210_3 for screening a lithium secondary battery material, according to one embodiment of the present disclosure. As illustrated, a plurality of user terminals 210_1, 210_2, and 210_3 may be connected to an information processing system 230 (e.g., the lithium secondary battery material screening system 120 of FIG. 1) that can provide a lithium secondary battery material screening service via a network 220. Here, the plurality of user terminals 210_1, 210_2, and 210_3 may include terminals of users who are recipients of the lithium secondary battery material screening service.

In an embodiment, the information processing system 230 may include one or more server devices and/or databases capable of storing, providing, and executing computer-executable programs (e.g., downloadable applications) and data related to providing a lithium secondary battery material screening service, or one or more distributed computing devices and/or distributed databases based on cloud computing services.

The lithium secondary battery material screening service provided by the information processing system 230 may be provided to users through a lithium secondary battery material screening application, a web browser, a web browser extension program, etc., installed on each of a plurality of user terminals 210_1, 210_2, and 210_3. For example, the information processing system 230 may provide information corresponding to a request for information about a target substance received from user terminals 210_1, 210_2, and 210_3 through the lithium secondary battery material screening application, etc., or perform processing corresponding thereto.

The plurality of user terminals 210_1, 210_2, and 210_3 may communicate with the information processing system 230 via a network 220. The network 220 may be configured to enable communication between the plurality of user terminals 210_1, 210_2, and 210_3 and the information processing system 230. Depending on the installation environment, the network 220 may be configured by, for example, a wired network such as Ethernet, a power line communication, a telephone line communication device, and RS-serial communication, a wireless network such as a mobile communication network, a wireless LAN (WLAN), Wi-Fi, Bluetooth, and ZigBee, or a combination thereof. The communication method is not limited, and may include not only a communication method utilizing a communication network (for example, a mobile communication network, a wired internet, a wireless internet, a broadcasting network, a satellite network, etc.) that may be included in the network 220, but also a short-range wireless communication between the user terminals 210_1, 210_2, and 210_3.

A mobile phone terminal 210_1, a tablet terminal 210_2, and a PC terminal 210_3 are illustrated as examples of the user terminals in FIG. 2, but the present disclosure is not limited thereto, and the user terminals 210_1, 210_2, and 210_3 may be any computing device capable of wired and/or wireless communication and capable of installing and executing a lithium secondary battery material screening application, a web browser, etc. For example, the user terminal may include an AI speaker, a smartphone, a mobile phone, a navigation device, a computer, a laptop, a digital broadcasting terminal, a personal digital assistant (PDA), a portable multimedia player (PMP), a tablet PC, a game console, a wearable device, an internet of things (IoT) device, a virtual reality (VR) device, an augmented reality (AR) device, a set-top box, etc. In addition, although FIG. 2 illustrates that three user terminals 210_1, 210_2, and 210_3 communicate with the information processing system 230 via the network 220, but the present disclosure is not limited thereto, and a different number of user terminals may be configured to communicate with the information processing system 230 via the network 220.

FIG. 2 illustrates a configuration in which a user's request (e.g., a request for information on a target substance) is transmitted to the information processing system 230 through the user terminals 210_1, 210_2, and 210_3, but the present disclosure is not limited thereto, and the user's request may be provided to the information processing system 230 through an input device associated with the information processing system 230 without passing through the user terminals 210_1, 210_2, and 210_3. The result of processing the user's request (e.g., information 130 on a target substance of FIG. 1) may be provided to the user through an output device (e.g., a display, etc.) associated with the information processing system 230.

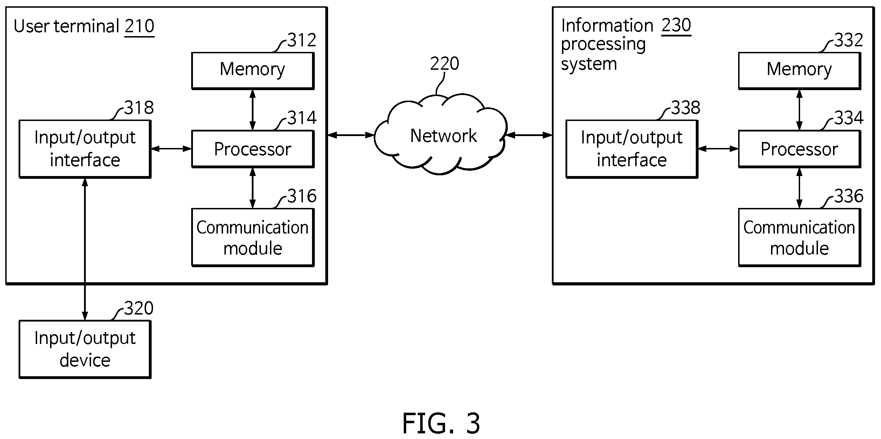

FIG. 3 is a block diagram showing an internal configuration of the user terminal 210 and the information processing system 230, according to an embodiment of the present disclosure. The user terminal 210 may refer to any computing device capable of executing an application, a web browser, etc., and capable of wired/wireless communication, and may include, for example, a mobile phone terminal 210_1, a tablet terminal 210_2, and a PC terminal 210_3 of FIG. 2. As illustrated, the user terminal 210 may include a memory 312, a processor 314, a communication module 316, and an input/output interface 318. Similarly, the information processing system 230 may include a memory 332, a processor 334, a communication module 336, and an input/output interface 338. As illustrated in FIG. 3, the user terminal 210 and the information processing system 230 may be configured to communicate information and/or data via the network 220 using their respective communication modules 316 and 336. In addition, the input/output device 320 may be configured to input information and/or data to the user terminal 210 or output information and/or data generated from the user terminal 210 via the input/output interface 318.

The memories 312 and 332 may include any non-transitory computer-readable recording medium. According to an embodiment, the memories 312 and 332 may include a permanent mass storage device such as a read only memory (ROM), a disk drive, a solid state drive (SSD), a flash memory, etc. As an example, the permanent mass storage device such as a ROM, an SSD, a flash memory, a disk drive, etc., may be included in the user terminal 210 or the information processing system 230 as a separate permanent storage device that is distinguished from the memory. In addition, an operating system and at least one program code may be stored in the memories 312 and 332.

These software components may be loaded from a computer-readable recording medium separate from the memories 312 and 332. Such separate computer-readable recording media may include recording media directly connectable to the user terminal 210 and the information processing system 230, for example, computer-readable recording media such as a floppy drive, a disk, a tape, a DVD/CD-ROM drive, a memory card, etc. As an example, software components may be loaded into the memories 312 and 332 via communication modules 316 and 336 other than a computer-readable recording medium. For example, at least one program may be loaded into the memories 312 and 332 based on a computer program that is installed by files provided by developers or a file distribution system distributing installation files of applications via a network 220.

The processors 314 and 334 may be configured to process instructions of a computer program by performing basic arithmetic, logic, and input/output operations. The instructions may be provided to the processors 314 and 334 by the memories 312 and 332 or the communication modules 316 and 336. For example, the processors 314 and 334 may be configured to execute instructions received according to program codes stored in a recording device such as the memories 312 and 332.

The communication modules 316 and 336 may provide a configuration or function for the user terminal 210 and the information processing system 230 to communicate with each other via the network 220, and may provide a configuration or function for the user terminal 210 and/or the information processing system 230 to communicate with another user terminal or another system (for example, a separate cloud system, etc.). For example, a request or data (for example, a request for providing information on a target substance, etc.) generated by the processor 314 of the user terminal 210 according to a program code stored in a recording device such as the memory 312 may be transmitted to the information processing system 230 via the network 220 under the control of the communication module 316. Conversely, a control signal or command provided under the control of the processor 334 of the information processing system 230 may be received by the user terminal 210 through the communication module 316 of the user terminal 210 via the communication module 336 and the network 220.

The input/output interface 318 may be a means for interfacing with an input/output device 320. As an example, the input device may include a device such as a camera, a keyboard, a microphone, a mouse, etc., including an audio sensor and/or an image sensor, and the output device may include a device such as a display, a speaker, a haptic feedback device, etc. As an example, the input/output interface 318 may be a means for interfacing with a device that has a configuration or function integrated into one for performing input and output, such as a touch screen, etc. For example, when the processor 314 of the user terminal 210 processes instructions of a computer program loaded into the memory 312, a service screen configured for using information and/or data provided by the information processing system 230 or another user terminal may be displayed on the display through the input/output interface 318. Although FIG. 3 illustrates that the input/output device 320 is not included in the user terminal 210, it is not limited thereto, and may be configured as a single device with the user terminal 210. In addition, the input/output interface 338 of the information processing system 230 may be a means for interfacing with a device (not shown) for input or output that may be connected to the information processing system 230 or may be included in the information processing system 230. Although FIG. 3 shows the input/output interfaces 318 and 338 as elements configured separately from the processors 314 and 334, the input/output interfaces 318 and 338 are not limited thereto, and may be configured to be included in the processors 314 and 334.

The user terminal 210 and the information processing system 230 may include additional components other than those shown in FIG. 3. However, it is not necessary to illustrate conventional components. In one embodiment, the user terminal 210 may be implemented to include at least some of the input/output devices 320 described above. In addition, the user terminal 210 may further include other components such as a transceiver, a global positioning system (GPS) module, a camera, various sensors, a database, etc. For example, if the user terminal 210 is a smartphone, it may include components generally included in the smartphone, for example, various components such as an acceleration sensor, a gyro sensor, a microphone module, a camera module, various physical buttons, a button using a touch panel, input/output ports, and a vibrator for vibration, all of which may be implemented to be further included in the user terminal 210.

While a program or application for a lithium secondary battery material screening service, etc., is in operation, the processor 314 may receive text, images, video, voice and/or motion, etc., as input or selected through an input device, such as a camera and microphone, including a touch screen, a keyboard, an audio sensor and/or an image sensor, connected to the input/output interface 318, and may store the received text, images, video, voice and/or motion, etc., in the memory 312 or provide any of them to the information processing system 230 through the communication module 316 and the network 220.

The processor 314 of the user terminal 210 may be configured to manage, process, and/or store information and/or data received from an input/output device 320, an additional user terminal, an information processing system 230, and/or a plurality of external systems. The information and/or data processed by the processor 314 may be provided to the information processing system 230 via the communication module 316 and the network 220. The processor 314 of the user terminal 210 may transmit information and/or data to the input/output device 320 via the input/output interface 318 and output the information and/or data. For example, the processor 314 may output or display the received information and/or data on a screen associated with the user terminal 210.

The processor 334 of the information processing system 230 may be configured to manage, process, and/or store information and/or data received from a plurality of user terminals 210 and/or a plurality of external systems. The information and/or data processed by the processor 334 may be provided to the user terminal 210 through the communication module 336 and the network 220.

In FIGS. 2 and 3, it is illustrated that the user terminal 210 communicates with the information processing system 230 that can provide a lithium secondary battery material screening service through the network, but it is not limited thereto, and the user can access or operate the information processing system 230 by using the input/output interface 338 provided by the information processing system 230 without going through the user terminal 210 and the network.

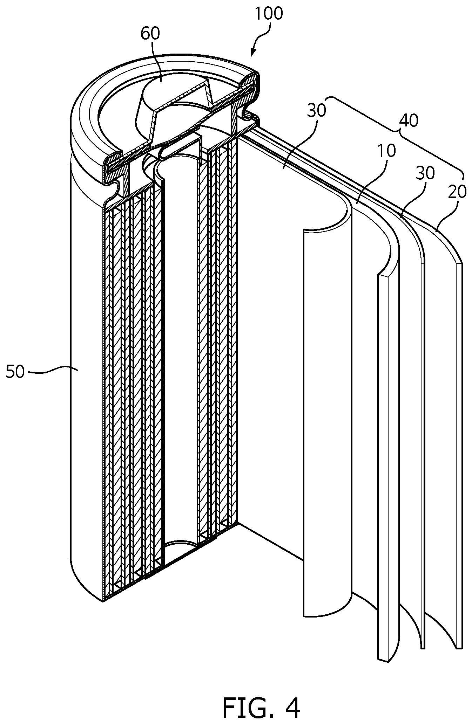

FIG. 4 is a cross-sectional diagram showing a lithium secondary battery 100, according to an embodiment of the present disclosure. Referring to FIG. 4, a lithium secondary battery 100 may include an electrode assembly 40 having a positive electrode 10, a negative electrode 20, and a separator 30 interposed therebetween, and a case 50 in which the electrode assembly 40 is housed within. The positive electrode 10, the negative electrode 20, and the separator 30 may be impregnated with an electrolyte (not shown). The lithium secondary battery 100 may include an electrode tab 60 that serves as an electrical path for inducing a current formed in the electrode assembly 40 to the exterior of the lithium secondary battery 100. Referring to FIG. 4, the shape of the lithium secondary battery has a cylindrical shape, but is not limited thereto. For example, the lithium secondary battery may have a prismatic type, a pouch type, a coin type, or the like.

The electrolyte solution for a rechargeable lithium battery may include a non-aqueous organic solvent and a lithium salt.

The non-aqueous organic solvent may serve as a medium for transmitting ions taking part in the electrochemical reaction of a battery.

The non-aqueous organic solvent may be a carbonate-based, an ester-based, an ether-based, a ketone-based, or an alcohol-based solvent, an aprotic solvent, or a combination thereof.

The carbonate-based solvent may include dimethyl carbonate (DMC), diethyl carbonate (DEC), dipropyl carbonate (DPC), methylpropyl carbonate (MPC), ethylpropyl carbonate (EPC), methylethyl carbonate (MEC), ethylene carbonate (EC), propylene carbonate (PC), butylene carbonate (BC), and the like.

The ester-based solvent may include methyl acetate, ethyl acetate, n-propyl acetate, dimethyl acetate, methyl propionate, ethyl propionate, decanolide, mevalonolactone, valerolactone, caprolactone, and the like.

The ether-based solvent may include dibutyl ether, tetraglyme, diglyme, dimethoxyethane, 2-methyltetrahydrofuran, 2,5-dimethyltetrahydrofuran, tetrahydrofuran, and the like.

The ketone-based solvent may include cyclohexanone, and the like.

The alcohol-based solvent may include ethanol, isopropyl alcohol, and the like.

The aprotic solvent may include nitriles such as R-CN (wherein R is a C₂ to C₂₀ linear, branched, or cyclic hydrocarbon group which may include a double bond, an aromatic ring, or an ether bond, and the like), amides such as dimethylformamide, dioxolanes such as 1,3-dioxolane, 1,4-dioxolane, and the like, and sulfolanes, etc.

The non-aqueous organic solvents may be used alone or in combination with two or more.

In addition, when using a carbonate-based solvent, a cyclic carbonate and a chain carbonate may be combined and used, and the cyclic carbonate and the chain carbonate may be combined in a volume ratio of about 1:1 to about 1:9.

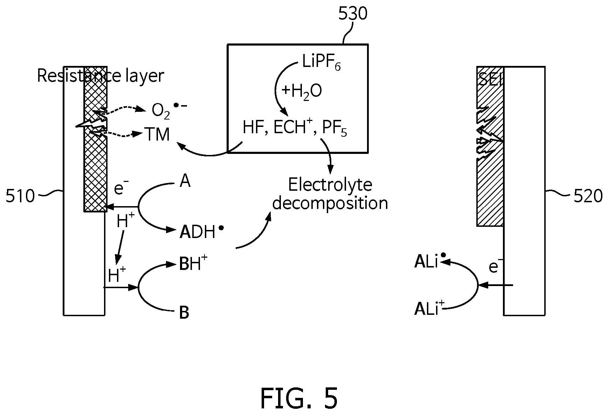

FIG. 5 is a schematic diagram showing deterioration of a lithium secondary battery including a positive electrode 510, a negative electrode 520, and an electrolyte, according to an embodiment of the present disclosure. FIG. 5 may refer to a factor (e.g., a reaction equation) that causes deterioration of the lithium secondary battery.

Referring to the positive electrode 510 illustrated in FIG. 5, an interfacial layer (a resistance layer as shown in FIG. 5) may be formed on the positive electrode 510. For example, a cathode electrode interface (CEI) layer may be formed on the interface of the positive electrode 510. The interfacial layer may have resistance. Accordingly, the secondary battery may be deteriorated by the resistance of the interfacial layer.

The interfacial layer formed on the positive electrode 510 may have a crack at which the positive electrode 510 is exposed. Oxygen radicals (O₂^{•-} as shown in FIG. 5) may be generated by the positive electrode 510 exposed through the crack of the interfacial layer, and transition metal ions (TM as shown in FIG. 5) may be generated. For example, the oxygen radicals and transition metal ions may be generated by the reaction between the positive electrode 510 and the electrolyte. The oxygen radicals and transition metal ions may cause a side reaction inside the secondary battery, thereby deteriorating the secondary battery.

The first material (A as shown in FIG. 5) included in the electrolyte may be oxidized at or around the positive electrode 510 and a dehydrogenation reaction may occur. The hydrogen ions generated by the dehydrogenation reaction may react with the second material (B as shown in FIG. 5) included in the electrolyte to cause a protonation reaction. The dehydrogenated first material (ADH^{•} as shown in FIG. 5) and the protonated second material (BH⁺ in FIG. 5) may cause decomposition of the electrolyte. As the secondary battery is repeatedly charged and discharged, additional dehydrogenated first material and protonated second material can be generated, and the additional dehydrogenated first material and protonated second material may further decompose the electrolyte. As the electrolyte, allowing lithium to migrate, becomes decomposed, the secondary battery may deteriorate.

Focusing on the negative electrode 520 shown in FIG. 5, a solid electrolyte interphase (SEI) layer may be formed on the interface of the negative electrode 520. Specifically, the SEI layer may be formed by a reduction reaction of a material included in the electrolyte (e.g., including an anion of a lithium salt) and/or a material included in the negative electrode 520 on the surface of the negative electrode 520. In addition, a lithium ion complex (ALi⁺ as shown in FIG. 5) in which lithium ions are combined with another material included in the electrolyte may undergo a reduction reaction at or around the negative electrode 520. A reduced lithium ion complex (ALi• in FIG. 5) may be generated by the reduction reaction. The negative electrode 520 may be deteriorated by taking away electrons at or around the negative electrode 520 by the reduction reaction.

Deterioration factors related to the electrolyte will be described based on an example where the positive electrode 510 of the lithium secondary battery includes lithium hexafluorophosphate (LiPF₆) and the electrolyte includes ethylene carbonate (EC), which is an organic solvent of the lithium secondary battery. Focusing on the electrolyte, the lithium hexafluorophosphate may react with water to produce HF, PF₅, ECH⁺ (protonated ethylene carbonate), etc. At least some of the products may react with transition metal ions or cause electrolyte decomposition. ECH⁺ may react with EC and H₂O, and the EC may be decomposed into acetylenediol (HOCCOH) and CO₂. As the secondary battery is continuously used, the decomposition of the EC and electrolyte may continue to occur through the above-described process, which may cause deterioration of the secondary battery.

Among the deterioration factors of the secondary battery described above based on the example of lithium hexafluorophosphate, it is necessary to search for a material capable of suppressing deterioration. Referring to FIG. 5, it is necessary to search for a material capable of suppressing deterioration related to the main deterioration reaction equation 530. Specifically, in the main deterioration reaction equation 530, as the organic substance included in the electrolyte reacts with lithium ions, the deterioration of the secondary battery including the electrolyte can be suppressed. In addition, as the organic substance included in the electrolyte reacts with the anions of the lithium salt (e.g., hexafluorophosphate ion), the deterioration of the secondary battery including the electrolyte can be suppressed. For example, as the organic substance included in the electrolyte interacts with the hexafluorophosphate ion more than a representative organic solvent (e.g., EC), which is an organic solvent of the lithium secondary battery, the deterioration of the secondary battery including the electrolyte can be suppressed. In addition, as the organic substance included in the electrolyte interacts with the hexafluorophosphate ion more than a representative organic solvent, at least some of the organic substances can form at least a SEI layer including the hexafluorophosphate ion. By forming the SEI layer, including anions of the lithium salt, the deterioration of the secondary battery can be suppressed, and high-temperature characteristics of the secondary battery can be improved. As an example, as the organic substance included in the electrolyte reacts with lithium ions more than a representative additive (e.g., fluoroethylene carbonate (FEC)), which is an electrolyte additive of the lithium secondary battery, the deterioration of the secondary battery including the electrolyte can be suppressed. The lithium secondary battery material screening system (e.g., the lithium secondary battery material screening system 120 of FIG. 1) may generate output information about a target substance (e.g., output information 130 about a target substance of FIG. 1), which is an organic substance for suppressing the deterioration related to the main deterioration reaction equation 530, by applying at least one filter to a database (e.g., material database 122 of FIG. 1).

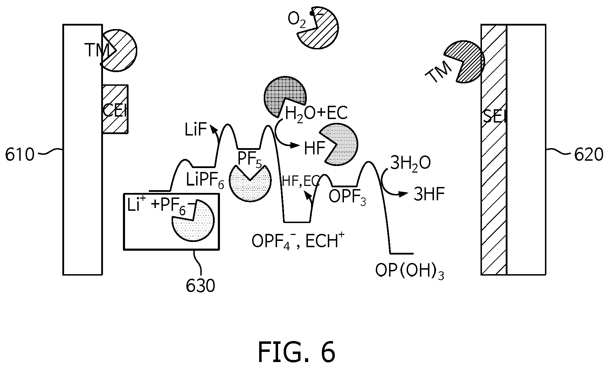

FIG. 6 is a schematic diagram showing a lithium secondary battery including a positive electrode 610, a negative electrode 620, and an electrolyte, for explaining the role of the electrolyte, according to an embodiment of the present disclosure.

Referring to the positive electrode 610 illustrated in FIG. 6, a CEI layer may be formed on the interface of the positive electrode 610. For example, an organic substance included in the electrolyte may be oxidized at or around the positive electrode 610 to form the CEI layer.

Transition metal (TM as shown in FIG. 6) ions that deteriorate the secondary battery at or around the positive electrode 610 may be combined with the organic substance contained in the electrolyte. As a result, the deterioration of the secondary battery can be suppressed.

Referring to the negative electrode 620 illustrated in FIG. 6, an SEI layer may be formed on the interface of the negative electrode 620. For example, an organic substance included in the electrolyte may be oxidized at or around the negative electrode 620 to form the SEI layer.

Transition metal ions that deteriorate the secondary battery at or around the negative electrode 620 may be combined with the organic substance contained in the electrolyte. As a result, the deterioration of the secondary battery can be suppressed.

The electrolyte may include oxygen radicals generated in the secondary battery. The oxygen radicals that contribute to the deterioration of the secondary battery may be combined with the organic substance contained in the electrolyte. As a result, the deterioration of the secondary battery can be suppressed.

The lithium salt included in the electrolyte can cause the deterioration of the secondary battery through various reaction paths. Deterioration factors related to the electrolyte will be described based on an example where the positive electrode 610 of the lithium secondary battery includes lithium hexafluorophosphate (LiPF₆) and the electrolyte includes ethylene carbonate (EC), which is an organic solvent of the lithium secondary battery. The electrolyte includes lithium hexafluorophosphate, which is a lithium salt, and may include lithium ions and hexafluorophosphate ions. The hexafluorophosphate ions may be combined with the organic substance included in the electrolyte. Phosphorus pentafluoride (PF₅) present in the reaction path of lithium hexafluorophosphate may be combined with the organic substance included in the electrolyte. Water, which may be involved in the reaction path of lithium hexafluorophosphate, may be combined with the organic substance included in the electrolyte. Hydrogen fluoride (HF), which may be involved in the reaction path of lithium hexafluorophosphate, may be combined with the organic substance included in the electrolyte. As a result, the deterioration of the secondary battery can be suppressed.

Among the roles of the organic substance included in the above-described electrolyte based on the example of lithium hexafluorophosphate, it is necessary to search for an organic substance that contributes to suppressing deterioration on the anionic side of the lithium salt. Referring to Fig. 6, in the main role 630 of the organic substance, as the organic substance included in the electrolyte combines with hexafluorophosphate ions, the deterioration of the secondary battery due to lithium hexafluorophosphate can be suppressed. For example, as the organic substance included in the electrolyte combines with the hexafluorophosphate ion more than a representative organic solvent (e.g., EC), which is an organic solvent of the lithium secondary battery, the deterioration of the secondary battery including the electrolyte can be suppressed. The lithium secondary battery material screening system (e.g., the lithium secondary battery material screening system 120 of FIG. 1) may generate output information about a target substance (e.g., output information 130 about a target substance of FIG. 1), which is an organic substance performing a main role 630 of an organic substance, by applying at least one filter to a database (e.g., a material database 122 of FIG. 1).

FIG. 7 and Table 1 show an example of information about a suitable organic substance, according to an embodiment of the present disclosure. The lithium secondary battery material screening system may generate a database by receiving information about the organic substance and storing information about the organic substance (e.g., information 110 about the organic substance of FIG. 1) based on a plurality of parameters.

**Table 1**

| Structure | NAME | SMILES | Type | Mass | Electron Affinity | Ionization Energy | Li⁺ Interaction | PF₆⁻ Interaction |
|---|---|---|---|---|---|---|---|---|
| 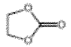 | EC | C1COC(=O)O1 | Neutral | 88.06 | -0.95 | 11.12 | -1.86 | -0.36 |
| 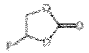 | FEC | C1C(OC(=O)O1)F | Neutral | 106.05 | -0.87 | 11.1 | -1.65 | -0.43 |
| 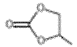 | PC | CC1COC(=O)O1 | Neutral | 102.09 | -0.82 | 10.51 | -1.92 | -0.38 |
| 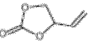 | VEC | O=C(O1)OCC1C=C | Neutral | 114.1 | 0.98 | 10.25 | -1.93 | -0.38 |
| 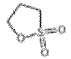 | PS | O=S1(=O)CCCO1 | Neutral | 122.14 | -0.73 | 10.7 | -1.82 | -0.37 |
| 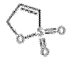 | PES | O=S1(C=CCO1)=O | Neutral | 120.13 | 0.15 | 10.61 | -1.81 | -0.46 |
| 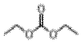 | DEC | CCOC(=O)OCC | Neutral | 118.13 | -1.28 | 10.36 | -1.92 | 0.12 |
| 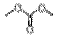 | DMC | COC(=O)OC | Neutral | 90.08 | -1.48 | 10.85 | -1.7 | 0.16 |
| 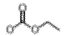 | EA | CCOC(=O)C | Neutral | 88.11 | -1.29 | 10.04 | -1.77 | 0.16 |
| 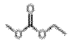 | EMC | CCOC(=O)OC | Neutral | 104.1 | -1.3 | 10.39 | -1.81 | 0.13 |
| 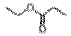 | EP | CCC(=O)OCC | Neutral | 102.13 | -1.25 | 9.94 | -1.76 | 0.16 |
| 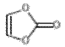 | VC | C1=COC(=O)O1 | Neutral | 86.05 | -1.14 | 9.69 | -1.7 | -0.24 |

Referring to FIG. 7 and Table 1, a suitable organic substance may include ethylene carbonate (EC), fluoroethylene carbonate (FEC), propylene carbonate (PC), vinylethylene carbonate (VEC), propene sultone (PS), propane sultone (PES), diethyl carbonate (DEC), dimethyl carbonate (DMC), ethyl acetate (EA), ethyl methyl carbonate (EMC), ethyl propionate (EP), vinylene carbonate (VC), etc. The information on the organic substance may include a compound name, SMILES, etc., as the name capable of specifying the organic substance. The information about the organic substance may include information corresponding to the molecular structure (Structure as shown in FIG. 7 and Table 1), the ion type of the organic substance (Type as shown in FIG. 7 and Table 1), the molecular weight (Mass as shown in FIG. 7 and Table 1), the electron affinity (Electron Affinity as shown in FIG. 7 and Table 1), the ionization energy (Ionization Energy as shown in FIG. 7 and Table 1), the lithium ion reaction energy (Li⁺ interaction as shown in FIG. 7 and Table 1), and the coordination energy of the organic substance and the hexafluorophosphate ion (PF₆⁻Interaction as shown in FIG. 7 and Table 1). Here, the unit of energy may be eV.

The plurality of parameters may include parameters related to the name of the compound, SMILES, the ion type, the molecular weight, the electron affinity, the ionization energy, the lithium ion reaction energy, and the coordination energy of the organic substance and the hexafluorophosphate ion, respectively. However, without limitation, the plurality of parameters may include more or less parameters than the parameters illustrated in FIG. 7 and Table 1, the organic substance may include more or less organic substances than the organic substance illustrated in FIG. 7 and Table 1, and the information about the organic substance may include information corresponding to a plurality of more or less parameters.

The lithium secondary battery screening system may receive (or generate) information about the organic substance described above and generate a database based on the information about the organic substance.

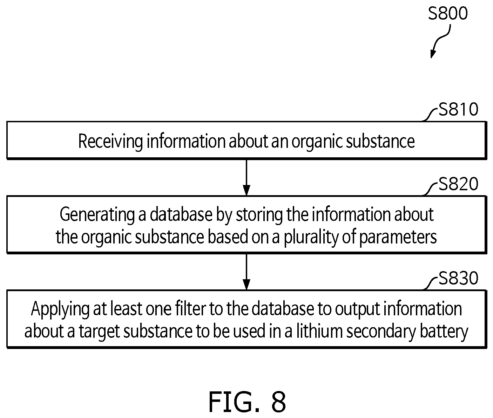

FIG. 8 is a flowchart showing an example of a lithium secondary battery material screening method S800, according to an embodiment of the present disclosure. The lithium secondary battery material screening method S800 may be performed by the lithium secondary battery material screening system (e.g., the lithium secondary battery material screening system 120 of FIG. 1). The lithium secondary battery material screening system may include a processor.

The lithium secondary battery material screening method S800 may be initiated by receiving information about an organic substance S810. In an embodiment, the processor may generate a database by storing the information about the organic substance based on a plurality of parameters S820. For example, the plurality of parameters may include parameters associated with at least one of an ion type, a molecular weight, an electron affinity, an ionization energy, a lithium ion (Li⁺) reaction energy, and a coordination energy between an organic substance and an anion of a lithium salt.

In an embodiment, the processor may apply at least one filter to the database to generate output information about a target substance configured to be used in a lithium secondary battery S830. Here, the target substance can suppress deterioration of the lithium secondary battery caused by the anion of the lithium salt.

In an embodiment, the at least one filter may include a first filter, wherein the first filter may filter out, among the materials included in the database, a material having an electron affinity greater than a predetermined electron affinity threshold. Here, the predetermined electron affinity threshold may be associated with an electron affinity of a representative organic solvent, which is an organic solvent of the lithium secondary battery. For example, the representative organic solvent may be one of ethylene carbonate (EC), fluoroethylene carbonate (FEC), ethylmethyl carbonate (EMC), dimethyl carbonate (DMC), ethyl propionate (EP), and ethyl acetate (EA).

In an embodiment, the at least one filter may include a second filter, wherein the second filter may filter out, among the materials included in the database, a material having an ionization energy greater than a predetermined ionization energy threshold. Here, the predetermined ionization energy threshold may be associated with an internal environment of the lithium secondary battery.

In an embodiment, the at least one filter may include a third filter, wherein the third filter may filter out, among the materials included in the database, a material having a lithium ion reaction energy less than a predetermined lithium ion reaction energy threshold.

In an embodiment, the lithium ion reaction energy threshold may be related to a reaction energy between a lithium ion and a representative additive, which can be an electrolyte additive of the lithium secondary battery. For example, the representative additive may be one of vinylene carbonate (VC) and fluoroethylene carbonate (FEC).

In an embodiment, the at least one filter may include a fourth filter, wherein the fourth filter may filter out, among the materials included in the database, a material having a coordination energy between the material included in the database and a hexafluorophosphate ion less than a predetermined hexafluorophosphate ion coordination energy threshold. The predetermined hexafluorophosphate ion coordination energy threshold may related to a coordination energy between a hexafluorophosphate ion and a representative organic solvent, which is an organic solvent of the lithium secondary battery.

In an embodiment, the processor may receive an input for selecting at least one of the plurality of parameters. The processor may receive information about a numerical range for the selected parameter. The at least one filter may include a fifth filter, wherein the fifth filter may filter out, among the materials included in the database, a material in which information related to at least one of the plurality of selected parameters of the materials included in the database is included in the numerical range.

In an embodiment, the processor may receive an input of selecting at least one of the plurality of parameters on a user interface. The processor may output information about at least one of the plurality of parameters selected among information about the target substance on the user interface.

The flowchart of FIG. 8 and the above description are merely examples of the present disclosure, and the scope of the present disclosure is not limited to the flowchart of FIG. 8 and the above description. For example, one or more steps in the flowchart and the above description may be added/changed/deleted, the order of one or more steps may be changed, and one or more steps may be performed simultaneously.

The above-described method may be provided as a computer program stored on a computer-readable recording medium for execution on a computer. The medium may continuously store the computer-executable program, or may temporarily store it for execution or download. In addition, the medium may be a variety of recording means or storage means in the form of a single hardware or a combination of multiple hardware, and is not limited to a medium directly connected to a computer system, and may be distributed on a network. Examples of media may include magnetic media such as hard disks, floppy disks, and magnetic tapes, optical recording media such as CD-ROM and DVD, magneto-optical media such as floptical disks, and those configured to store program instructions, including ROM, RAM, and flash memory, etc. In addition, examples of other media may include recording media or storage media managed by app stores that distribute applications, or other sites, servers, etc., that supply or distribute various software.

The methods, operations, or techniques of the present disclosure may be implemented by various means. For example, these techniques may be implemented in hardware, firmware, software, or a combination thereof. Those skilled in the art will appreciate that the various example logical blocks, modules, circuits, and algorithm steps described in connection with the present disclosure may be implemented in electronic hardware, computer software, or combinations of both. To clearly illustrate this interchangeability of hardware and software, various example components, blocks, modules, circuits, and steps have been described above generally in terms of their functionality. Whether such functionality is implemented as hardware or software will depend upon the design requirements imposed on a particular application and the overall system. Those skilled in the art may implement the described functionality in various ways for each particular application, but such implementations should not be construed as departing from the scope of the present disclosure.

In a hardware implementation, the processing units utilized to perform the techniques may be implemented in one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described herein, a computer, or a combination thereof.

Accordingly, the various example logical blocks, modules, and circuits described in connection with the present disclosure may be implemented or performed by any combination of a general-purpose processor, a DSP, an ASIC, an FPGA or other programmable logic device, discrete gate or transistor logic, discrete hardware components, or those designed to perform the functions described herein. The general-purpose processor may be a microprocessor, but alternatively, the processor may be any conventional processor, controller, microcontroller, or state machine. The processor may also be implemented as a combination of computing devices, for example, a combination of a DSP and a microprocessor, a plurality of microprocessors, one or more microprocessors in conjunction with a DSP core, or any other components.

In firmware and/or software implementations, the techniques may be implemented as instructions stored on a computer-readable medium, such as random access memory (RAM), read-only memory (ROM), non-volatile random access memory (NVRAM), programmable read-only memory (PROM), erasable programmable read-only memory (EPROM), electrically erasable PROM (EEPROM), flash memory, a compact disc (CD), a magnetic or optical data storage device, etc. The instructions may be executable by one or more processors and may cause the processor(s) to perform certain aspects of the functionality described in the present disclosure.

When implemented in software, the techniques described above may be stored on a computer-readable medium as one or more instructions or code or transmitted via a computer-readable medium. The computer-readable media may include computer storage media and communication media, including any medium that facilitates transfer of a computer program from one place to another. The storage media may be any available media that can be accessed by a computer. As a nonlimiting example, such computer-readable media can include RAM, ROM, EEPROM, CD-ROM or other optical disk storage, magnetic disk storage or other magnetic storage devices, or any other medium that can be used to carry or store desired program code in the form of instructions or data structures and can be accessed by a computer. In addition, any connection is appropriately referred to as a computer-readable medium.

For example, if the software is transmitted from a website, server, or other remote source using coaxial cable, fiber optic cable, twisted pair, digital subscriber line (DSL), or wireless technologies such as infrared, radio, and microwave, then the coaxial cable, fiber optic cable, twisted pair, digital subscriber line, or wireless technologies such as infrared, radio, and microwave, are included within the definition of media. The disk and disc as used herein includes compact discs (CDs), laser discs, optical discs, digital versatile discs (DVDs), floppy discs, and Blu-ray discs, wherein the disks typically reproduce data magnetically, whereas the discs reproduce data optically using a laser. Combinations of the above should also be included within the scope of computer-readable media.

A software module may reside in RAM memory, flash memory, ROM memory, EPROM memory, EEPROM memory, registers, hard disks, removable disks, CD-ROMs, or any other known form of storage medium. An example storage medium may be coupled to the processor such that the processor can read information from the storage medium or write information on the storage medium. Alternatively, the storage medium may be integrated into the processor. The processor and the storage medium may exist within an ASIC. The ASIC may exist within a user terminal. Alternatively, the processor and the storage medium may exist as separate components in the user terminal.

While the embodiments described above have been described as utilizing aspects of the presently disclosed subject matter in one or more standalone computer systems, the present disclosure is not limited thereto, and may be implemented in conjunction with any computing environment, such as a network or distributed computing environment. Furthermore, aspects of the presently disclosed subject matter may be implemented in a plurality of processing chips or devices, and storage may be similarly affected across in a plurality of devices. Such devices may include PCs, network servers, and portable devices.

FIG. 9 is a schematic diagram showing generating output information 940 about a target substance using a plurality of filters 930 according to one embodiment of the present disclosure. A lithium secondary battery material screening system may include a database 910 and a plurality of filters 930. The lithium secondary battery material screening system may apply at least one filter included in the plurality of filters 930 to data included in the database 910 to generate output information 940 about a target substance. Here, in a lithium secondary battery including a target substance, the target substance is configured to suppress deterioration of the lithium secondary battery, for example, deterioration caused by the anion of the lithium salt.

The database 910 may include information about an organic substance. For example, the database 910 may include a name capable of specifying an organic substance, molecular characteristics and chemical characteristics of the organic substance, etc. In addition, the database 910 may include information necessary to generate information about the molecular characteristics and chemical characteristics of the organic substance. The information required to generate information about the molecular characteristics and chemical characteristics of the organic substance may be determined in advance or may be received in advance before applying a plurality of filters 930.

Material data 920 may be filtered by applying at least some of the plurality of filters 930, thereby generating output information 940 about a target substance. The material data 920 may be at least some of the data included in the database 910. For example, a first filter 931, a second filter 932, a third filter 933, and a fourth filter 934 may be applied to the material data 920 to generate the information 940 about a target substance. As an example, a fifth filter 935 may be applied to the material data 920 to generate the information 940 about a target substance. The filtering may be performed on the material.

The plurality of filters 930 may include the first filter 931 to the fifth filter 935. Referring to FIG. 9, five filters are illustrated, but the present disclosure is not limited thereto. For example, the plurality of filters 930 may include less or more than five filters.

In an embodiment, the first filter 931 may filter out a material included in the material data 920 having an electron affinity greater than an electron affinity threshold (e.g., a predetermined electron affinity threshold). The electron affinity threshold may be associated with an electron affinity of a representative organic solvent which is an organic solvent of a lithium secondary battery. For example, the representative organic solvent may be one of ethylene carbonate (EC), fluoroethylene carbonate (FEC), ethylmethyl carbonate (EMC), dimethyl carbonate (DMC), ethyl propionate (EP), and ethyl acetate (EA). The electron affinity threshold may be a value that adds or subtracts a value of 0.1 eV or less to the electron affinity of the representative organic solvent. For example, when the representative organic solvent is EC, the electron affinity of the representative organic solvent may be -0.95 eV. In this case, the electron affinity threshold may be one of -1.05 eV to -0.85 eV.

In an embodiment, the second filter 932 may filter out a material included in the material data 920 having an ionization energy greater than an ionization energy threshold (e.g., a predetermined ionization energy threshold). The ionization energy threshold may be associated with an internal environment of a lithium secondary battery. For example, if the ionization energy of any material inside the lithium secondary battery is about 10 eV or more, the material may be stable against oxidation inside the lithium secondary battery. In this case, the ionization energy threshold may be about 10 eV.

In an embodiment, the third filter 933 may filter out a material included in the material data 920 having a lithium ion reaction energy less than a lithium ion reaction energy threshold (e.g., a predetermined lithium ion reaction energy threshold). The lithium ion reaction energy threshold may be associated with a reaction energy between a lithium ion and a representative additive which is an additive of the lithium secondary battery. For example, the representative additive may be one of vinylene carbonate (VC) and fluoroethylene carbonate (FEC).The lithium ion reaction energy threshold may be a value that adds or subtracts a value of 0.1 eV or less to the lithium ion reaction energy of the representative additive. For example, when the representative additive is FEC, the lithium ion reaction energy of the representative additive may be -1.65 eV. In this case, the lithium ion reaction energy threshold may be one of -1.75 eV to -1.55 eV.

In an embodiment, the fourth filter 934 may filter out a material included in the material database 920 having a coordination energy between the material and a hexafluorophosphate ion less than a hexafluorophosphate ion coordination energy threshold (e.g., a predetermined hexafluorophosphate ion coordination energy threshold). The hexafluorophosphate ion coordination energy threshold may be associated with the coordination energy between the hexafluorophosphate ion and the representative organic solvent. The hexafluorophosphate ion coordination energy threshold may be a value that adds or subtracts a value of 0.1 eV or less to the hexafluorophosphate ion coordination energy of the representative organic solvent. For example, when the representative organic solvent is EC, the coordination energy between the representative organic solvent and the hexafluorophosphate ion may be -0.36 eV. In this case, the hexafluorophosphate ion coordination energy threshold may be one of -0.46 eV to -0.26 eV.

In an embodiment, the lithium secondary battery material screening system may receive a first input for selecting at least one of a plurality of parameters. Additionally, the lithium secondary battery material screening system may receive information about a first numerical range for the selected parameter (or parameters). The lithium secondary battery material screening system may generate a fifth filter 1035 based on the first input for selecting at least one of the plurality of parameters and the information about the first numerical range. In response to information related to an parameter selected by the first input among information about a material included in the material data 1020 being included in the first numerical range, the fifth filter 1035 may filter out the corresponding material.

Additionally, the lithium secondary battery material screening system may receive a second input for selecting at least one of a plurality of parameters. In addition, the lithium secondary battery material screening system may receive information about a second numerical range for the selected parameter (or parameters). After the fifth filter 935 is generated, the lithium secondary battery material screening system may further generate a sixth filter based on the second input for selecting at least one of the plurality of parameters and the information about the second numerical range. Thereafter, at least one of the fifth filter 935 or the sixth filter may be applied to the material data 920. That is, a plurality of filters generated by the user's input are generated, and the plurality of filters generated by the user's input may be applied to the material data 920.

In an embodiment, the filter applied to the material data 920 may be determined in advance before filtering is performed. For example, the lithium secondary battery material screening system may receive an input of selecting a filter for performing filtering. The lithium secondary battery material screening system may determine a filter to be applied to the material data 920 based on an input of selecting a filter. For example, the lithium secondary battery material screening system may receive an input for selecting a second filter 932 to a fourth filter 934, and determine the second filter 932 to the fourth filter 934 as filters to be applied to the material data 920.

In an embodiment, when at least two of the plurality of filters 930 are applied to the material data 920, AND or OR logic may be applied. For example, when the first filter 931 and the second filter 932 are applied to the material data 920, a material filtered by the first filter 931 and filtered by the second filter 932 may be determined as a target substance. Alternatively, when the first filter 931 and the second filter 932 are applied to the material data 920, a material filtered by the first filter 931 and a material filtered by the second filter 932 may be determined as a target substance. Whether to apply the AND or OR logic may be determined in advance, or information related thereto may be received (e.g., an input is received through a user interface).

In an embodiment, the lithium secondary battery material screening system may determine the filtered material as a target substance. The lithium secondary battery material screening system may output information about the filtered material as information 940 about the target substance. For example, the information 940 about the target substance may be outputted to a user terminal, etc., and the information 940 about the target substance may be outputted on a user interface of the user terminal.

In an embodiment, the lithium secondary battery material screening system may generate output information 940 about the target substance by applying the first filter 931 to the fourth filter 934 to the material data 920. For example, the material filtered by applying the first filter 931 to the fourth filter 934 may be VEC, PES, PS, PC, etc. The lithium secondary battery material screening system may output information about VEC, PES, PS, PC, etc. as output information 940 about the target substance.

As described above, a material that suppresses the deterioration of the lithium secondary battery caused by an anion of a lithium salt among organic substances may be screened using a plurality of filters 930. For example, an organic substance that is more easily reduced with the negative electrode than the representative organic solvent may be filtered using the first filter 931. An organic substance that has oxidation stability inside the lithium secondary battery may be filtered using the second filter 932. An organic substance that forms a coordination bond with lithium ions better than the representative additive may be filtered using the third filter 933. An organic substance capable of forming an SEI layer together with inorganic ions included in the electrolyte may be filtered using the fourth filter 934. Additionally, a suitable organic substance capable of further suppressing the deterioration of the lithium secondary battery may be searched by a user selecting at least one of a plurality of parameters and inputting a numerical range for the selected parameter (or parameters) to generate a filter (e.g., the fifth filter 935).

FIG. 10 is a schematic diagram showing an example of a user interface having output information about a target substance, according to an embodiment of the present disclosure. The user interface 1000 may display output information about a target substance. Referring to FIG. 10, the user interface 1000 may include a graphical interface 1020 on which information about a target substance can be displayed. For example, the target substance may include EC, EMC, and FEC. The graphical interface 1020 may display a graph corresponding to each of EC, EMC, and FEC.

In an embodiment, the graphical interface 1020 may display information about the target substance. For example, the graphical interface 1020 may display information corresponding to electric dipole (Dipole Moment on the graphical interface 1020 of FIG. 10), electron affinity (Electron Affinity on the graphical interface 1020 of FIG. 10), hexafluorophosphate ion coordination energy (PF₆⁻ Interaction on the graphical interface 1020 of FIG. 10), ionization energy (Ionization Energy on the graphical interface 1020 of FIG. 10), and lithium ion reaction energy (Li⁺ Interaction on the graphical interface 1020 of FIG. 10) among the information about the target substance. However, the type of information about the target substance is not limited thereto, and at least some of the information about the target substance may be displayed.

In an embodiment, the type of information displayed on the graphical interface 1020 may be determined by input received through a graphical control interface 1010. The type of information displayed may correspond to at least some of a plurality of parameters (e.g., a plurality of parameters that are the basis for generating a database). For example, the electric dipole, lithium ion reaction energy, ionization energy, hexafluorophosphate ion coordination energy, and electron affinity may be determined as the types of information displayed by input on the graphical control interface 1010. In addition, the type of graph (e.g., radial chart, bar chart, etc.) may be determined through the graphical control interface 1010.

FIG. 11 is a schematic diagram showing an example of a filter input interface 1100, according to an embodiment of the present disclosure. Based on an input received through the filter input interface 1100, the lithium secondary battery material screening system may generate a filter (e.g., the fifth filter 935 of FIG. 9).

In an embodiment, the filter input interface 1100 may include a parameter selection interface 1110 and a numeric input interface 1120. At least some of the plurality of parameters (e.g., a plurality of parameters that are the basis for generating a database) may be selected through the parameter selection interface 1110. In this case, the filter input interface 1100 may further include an input window for searching for parameters to select at least some of the plurality of parameters.

In an embodiment, the numeric input interface 1120 may display an input window associated with the selected parameter (or parameters). Referring to FIG. 11, the dipole moment, LUMO, HOMO, and hexafluorophosphate ion coordination energy may be selected and the corresponding parameters may be displayed on the numeric input interface 1120. A numerical range for the corresponding parameters may be input through the numeric input interface 1120. However, if there is a parameter (e.g., an ion type) that is not related to a numerical value among a plurality of parameters and the parameter is selected, the selected parameter may not be displayed on the numeric input interface 1120, or an input window for the selected parameter may be deactivated.

In an embodiment, the lithium secondary battery material screening system may generate a plurality of filters based on information about a selected parameter (or parameters) and a numerical range. For example, a first filter may be generated based on information about a selected first parameter and a first numerical range for the first parameter. In addition, a second filter may be generated based on information about a selected second parameter and a second numerical range for the second parameter. The logic of AND or OR may be applied to the first filter and the second filter. For example, a material filtered by the first filter and the second filter may be generated as output including a target substance. As another example, a material filtered by the first filter and a material filtered by the second filter may be determined as target substances.

As described with reference to FIGS. 10 and 11, a filter can be created through a user interface, and a target substance capable of suppressing deterioration of a lithium secondary battery can be searched using the created filter. Information about the target substance can be displayed with high readability through the user interface. A more suitable substance can be selected by a user comparing substances included in the target substances with each other through the user interface.

Although the present disclosure has been described above with respect to embodiments thereof, the present disclosure is not limited thereto. Various modifications and variations can be made thereto by those skilled in the art within the scope of the present disclosure and the equivalent scope of the appended claims.

Embodiments are set out in the following clauses:
Clause 1. An information processing system comprising:
   a communication module;
   a memory; and
   at least one processor connected to the memory and configured to execute at least one computer-readable program comprised in the memory,
   wherein the at least one program comprises instructions for:
      receiving information about one or more organic substances,
      generating a database by storing the information about the one or more organic substances based on a plurality of parameters, and
      applying at least one filter to the database to generate output information about one or more target substances configured to be used in a lithium secondary battery, the one or more target substances selected from the or more organic substances,
      wherein at least one of the one or more target substances is configured to form a solid electrolyte interphase (SEI) layer comprising an anion of a lithium salt.
Clause 2. The information processing system of clause 1, wherein the at least one filter comprises a first filter,
   wherein the first filter filters out a candidate material having an electron affinity greater than a predetermined electron affinity threshold.
Clause 3. The information processing system of clause 1 or clause 2, wherein the at least one filter comprises a second filter,
   wherein the second filter filters out a candidate material having an ionization energy greater than a predetermined ionization energy threshold.
Clause 4. The information processing system of any of clauses 1-3, wherein the at least one filter comprises a third filter,
   wherein the third filter filters out a candidate material having a lithium ion reaction energy less than a predetermined lithium ion reaction energy threshold.
Clause 5. The information processing system of any of clauses 1 to 4, wherein the at least one filter comprises a fourth filter,
   wherein the fourth filter filters out a candidate material having a coordination energy between the candidate material and a hexafluorophosphate ion (PF₆⁻) less than a predetermined hexafluorophosphate ion coordination energy threshold.

## Claims

1. A method for screening a material configured to be used in a lithium secondary battery, comprising:
receiving information about one or more organic substances (S810);
generating a database (910) by storing the information about the one or more organic substances based on a plurality of parameters (S820); and
applying at least one filter (930) to the database (910) to generate output information about one or more target substances (940) configured to be used in the lithium secondary battery (100), the one or more target substances selected from the one or more organic substances (S830),
wherein at least one of the one or more target substances is configured to form a solid electrolyte interphase (SEI) layer comprising an anion of a lithium salt.

2. The method as claimed in claim 1, wherein the plurality of parameters comprises an ion type, a molecular weight, an electron affinity, an ionization energy, a lithium ion (Li⁺) reaction energy, and/or a coordination energy between the one or more organic substances and an anion of a lithium salt.

3. The method as claimed in claim 1 or claim 2, wherein the at least one filter 930 comprises a first filter (931),
wherein the first filter filters (931) out a candidate material having an electron affinity greater than a predetermined electron affinity threshold.

4. The method as claimed in claim 3, wherein the predetermined electron affinity threshold is related to an electron affinity of an organic solvent previously determined to be used in the lithium secondary battery (100).

5. The method as claimed in any preceding claim, wherein the at least one filter (930) comprises a second filter (932),
wherein the second filter (932) filters out a candidate material having an ionization energy greater than a predetermined ionization energy threshold.

6. The method as claimed in claim 5, wherein the predetermined ionization energy threshold is related to an internal environment of the lithium secondary battery (100).

7. The method as claimed in any preceding claim, wherein the at least one filter (930) comprises a third filter,
wherein the third filter (933) filters out a candidate material having a lithium ion reaction energy less than a predetermined lithium ion reaction energy threshold.

8. The method as claimed in claim 7, wherein the predetermined lithium ion reaction energy threshold is related to a reaction energy between a lithium ion and an electrolyte additive previously determined to be used in the lithium secondary battery (100).

9. The method as claimed in any preceding claim, wherein the at least one filter (930) comprises a fourth filter (934),
wherein the fourth filter (934) filters out a candidate material having a coordination energy between the candidate material and a hexafluorophosphate ion (PF₆⁻) less than a predetermined hexafluorophosphate ion coordination energy threshold.

10. The method as claimed in claim 9, wherein the predetermined hexafluorophosphate ion coordination energy threshold is related to a coordination energy between the hexafluorophosphate ion and an organic solvent previously determined to be used in the lithium secondary battery (100).

11. The method as claimed in any of claims 4-10, wherein the organic solvent comprises ethylene carbonate (EC), fluoroethylene carbonate (FEC), ethylmethyl carbonate (EMC), dimethyl carbonate (DMC), ethyl propionate (EP), or ethyl acetate (EA).

12. The method as claimed in any of claims 8-11, wherein the electrolyte additive comprises vinylene carbonate (VC) or fluoroethylene carbonate (FEC).

13. The method as claimed in any preceding claim, further comprising:
receiving an input for selecting at least one of the plurality of parameters; and
receiving information about a numerical range for the at least one of the plurality of parameters,
wherein the at least one filter (930) comprises a fifth filter (935), and
wherein the fifth filter (935) filters out a candidate material in which the numerical range comprises information related to the at least one of the plurality of parameters.

14. The method as claimed in any preceding claim, wherein the generating of the output information comprises:
receiving an input of selecting at least one of the plurality of parameters on a user interface; and
generating output information about the at least one of the plurality of parameters selected from the output information about the one or more target substances on the user interface (1000).

15. An information processing system comprising:
a communication module (336);
a memory (332); and
at least one processor 334 connected to the memory (332) and configured to execute at least one computer-readable program comprised in the memory (332),
wherein the at least one program comprises instructions for:
receiving information about one or more organic substances (S810),
generating a database (910) by storing the information about the one or more organic substances based on a plurality of parameters (S820), and
applying at least one filter (930) to the database (910) to generate output information about one or more target substances (940) configured to be used in a lithium secondary battery (100), the one or more target substances selected from the or more organic substances (S830),
wherein at least one of the one or more target substances is configured to form a solid electrolyte interphase (SEI) layer comprising an anion of a lithium salt.
